# EUROPÄISCHE PATENTSCHRIFT

(11) **EP 0 729 937 B1**
(45) Veröffentlichungstag und Bekanntmachung des Hinweises auf die Patenterteilung: **28.04.1999**
(21) Anmeldenummer: 96102839.6
(22) Anmeldetag: 26.02.1996
(51) Int. Cl.: C07C 209/48, C07C 211/36

(54) **Verfahren zur Herstellung von 3-Aminomethyl-3,5,5-trimethylcyclohexylamin**
Process for the preparation of 3-aminomethyl-3,5,5-trimethylcyclohexylamine
Procédé pour la préparation de la 3-aminométhyl-3,5,5-triméthylcyclohexylamine

(30) Priorität: 03.03.1995 DE 19507398
(43) Veröffentlichungstag der Anmeldung: 04.09.1996
(73) Patentinhaber: BASF AKTIENGESELLSCHAFT, 67056 Ludwigshafen (DE)
(72) Erfinder: Voit, Guido, Dr., 69198 Schriesheim (DE); Witzel, Tom, Dr., 67069 Ludwigshafen (DE); Breitscheidel, Boris, Dr., 36043 Fulda (DE); Harder, Wolfgang, Dr., 69469 Weinheim (DE); Luyken, Hermann, 67069 Ludwigshafen (DE); Paul, Axel, Dr., 68623 Lampertheim (DE); Ross, Karl-Heinz, Dr., 67269 Grünstadt (DE); Wahl, Peter, Dr., 68526 Ladenburg (DE)

(56) Entgegenhaltungen:
- EP-A- 0 449 089
- EP-A- 0 503 246
- EP-A- 0 534 449
- EP-A- 0 636 409
- EP-A- 0 659 733

## Beschreibung

Die vorliegende Erfindung betrifft ein Verfahren zur Herstellung von 3-Aminomethyl-3,5,5-trimethylcyclohexylamin (Isophorondiamin, IPDA) durch Umsetzung von 3-Cyano-3,5,5-trimethylcyclohexanon (Isophoronnitril, IPN) mit Ammoniak und Wasserstoff in drei räumlich voneinander getrennten Reaktionsräumen.

Aus der EP-A-449 089 ist ein Verfahren zur Herstellung von IPDA aus IPN bekannt. Man setzt in einer ersten Stufe IPN mit Ammoniak an aciden Metalloxiden zum Isophoronnitril-imin um und hydriert dann in einem zweiten Schritt nach Zugabe von Wasserstoff an bekannten Hydriermetallen, bevorzugt Cobalt und/oder Ruthenium auf basischen Trägern oder in Gegenwart von basischen Komponenten, wie Alkali- oder Erdalkalihydroxiden, zu IPDA.

Das IPDA fällt in zwei stereoisomeren Formen an, wobei die Aminofunktion in 1-Position und die Aminomethyl-Funktion in 3-Position relativ zueinander cis oder trans stehen können (cis-IPDA und trans-IPDA).

IPDA findet z.B. Einsatz als Epoxidharzhärter oder - über das Isophorondiisocyanat - als Polyurethankomponente. Es gibt spezielle Anwendungsgebiete, in denen das cis/trans- Isomerenverhältnis von Bedeutung ist, wobei häufig ein cis-Anteil von z. B. > 67 % gewünscht wird.

Das in EP-A-449 089 beschriebene Verfahren ermöglicht die Herstellung von IPDA aus IPN mit - im Vergleich zu vorbeschriebenen Verfahren - hoher Raum-Zeit-Ausbeute und hoher chemischer Ausbeute. So sind bei Einsatz von Aluminiumoxid oder Titandioxid in der Iminierung und hochaktiven Cobalt-Katalysatoren für die Hydrierung, wie sie z.B. in DE-A-43 25 847 beschrieben sind, Ausbeuten an IPDA bis 98 % erreichbar, der cis-Anteil liegt dabei jedoch nur bei 60 %. Eine Steigerung des cis-Anteils auf z. B. 68 % ist möglich, aber nur unter Ausbeuteverlust.

Gesucht war daher nach einem verbesserten Verfahren, daß sowohl hohe Raum-Zeit-Ausbeute und hohe chemische Ausbeuten als auch einen erhöhten Anteil an cis-IPDA ermöglicht.

Demgemäß wurde ein neues und verbessertes Verfahren zur Herstellung von 3-Aminomethyl-3,5,5-trimethyl-cyclohexylamin aus 3-Cyano-3,5,5-trimethyl-cyclohexanon gefunden, welches dadurch gekennzeichnet ist, daß man in drei räumlich voneinander getrennten Reaktionsräumen
a) das 3-Cyano-3,5,5-trimethyl-cyclohexanon in einem ersten Reaktionsraum mit überschüssigem Ammoniak an aciden Metalloxidkatalysatoren bei Temperaturen von 20 bis 150°C und Drücken von 50 bis 300 bar umsetzt,
b) die entstandenen Reaktionsprodukte in einem zweiten Reaktionsraum mit Wasserstoff in Gegenwart von überschüssigem Ammoniak an Hydrierkatalysatoren gegebenenfalls mit basischen Komponenten oder auf neutralen oder basischen Trägern bei Temperaturen von 50 bis 100°C und Drücken von 50 bis 300 bar hydriert und
c) die entstandenen Reaktionsprodukte in einem dritten Reaktionsraum in Gegenwart von Wasserstoff an Hydrierkatalysatoren gegebenenfalls mit basischen Komponenten oder auf neutralen oder basischen Trägern bei Temperaturen von 110 bis 160°C und Drücken von 150 bis 300 bar hydriert.

Das erfindungsgemäße Verfahren läßt sich wie folgt durchführen:
a) In einer ersten Verfahrensstufe setzt man 3-Cyano-3,5,5-trimethyl-cyclohexanon mit überschüssigem Ammoniak bei Temperaturen von 20 bis 150°C, vorzugsweise von 30 bis 130°C, besonders bevorzugt von 50 bis 100°C und Drücken von 50 bis 300 bar, vorzugsweise von 100 bis 250 bar, zu 3-Cyano-3,5,5-trimethyl-cyclohexanonimin um.
   Als acide Metalloxidkatalysatoren eignen sich Aluminiumoxid, Siliciumdioxid, Titandioxid, Zirkondioxid, bevorzugt verwendet man Aluminiumoxid, Titandioxid, Zirkondioxid, insbesondere Aluminiumoxid und Titandioxid.
   Bei der Iminierung hält man eine Katalysatorbelastung von 0,01 bis 10, vorzugsweise von 0,05 bis 7, besonders bevorzugt von 0,1 bis 5 kg 3-Cyano-3,5,5-trimethylcyclohexanon pro kg Katalysator und Stunde ein. Pro Mol 3-Cyano-3,5,5-trimethylcyclohexanon setzt man bei der Iminierung zweckmäßig aber nicht zwingend 5 bis 500 Mol NH₃ bevorzugt 10 bis 400 Mol,besonders bevorzugt 20 bis 300 Mol ein. Die Iminierung des 3-Cyano-3,5,5-trimethyl-cyclohexanons kann auch in Anwesenheit eines Lösungsmittels, wie z.B. Alkanolen oder Tetrahydrofuran durchgeführt werden, bevorzugt arbeitet man aber ohne Zusatz eines Lösungsmittels.
   Die Iminierung wird bevorzugt kontinuierlich durchgeführt, z.B. in Druckbehältern oder Druckbehälterkaskaden. Nach einer besonders bevorzugten Ausführungsform werden 3-Cyano-3,5,5-trimethylcyclohexanon und NH₃ durch einen Rohrreaktor geleitet, in dem der Iminierungskatalysator in Form eines festen Bettes angeordnet ist.
b) Das so erhaltene Produkt wird in einer zweiten Stufe mit 3 bis 10.000 Moläquivalent Wasserstoff, bevorzugt 4,5 bis 100, gegebenenfalls nach Zufuhr von weiterem Ammoniak einer katalytischen Hydrierung unterzogen.
   Bei der Hydrierung hält man eine Temperatur von 60 bis 100°C und einen Druck von 50 bis 300 bar, bevorzugt von 100 bis 250 bar ein.
   Die Katalysatorbelastungen liegen zweckmäßig im Bereich von 0,01 bis 5 kg/[kgxh], bevorzugt bei 0,02 bis 2,5, besonders bevorzugt bei 0,05 bis 2 kg/[kgxh].
   Die Hydrierung wird vorzugsweise in flüssigem Ammoniak durchgeführt. Pro Mol 3-Cyano-3,5,5-trimethyl-cyclohexanonimin setzt man 5 bis 500 Mol NH₃ ein, bevorzugt 10 bis 400 Mol, besonders bevorzugt 20 bis 300 Mol. Zweckmäßigerweise wählt man mindestens das NH₃-Angebot, das bei der vorgelagerten Herstellung der 3-Cyano-3,5,5-trimethylcyclohexanonimin aus dem entsprechenden 3-Cyano-3,5,5-trimethylcyclohexanon eingestellt wurde. Der NH₃-Anteil kann jedoch auch vor der Hydrierung durch Zugabe von zusätzlichem NH₃ auf den gewünschten Wert erhöht werden.
   Die aminierende Hydrierung von 3-Cyano-3,5,5-trimethyl-cyclohexanonimin führt man bevorzugt kontinuierlich z.B. in druckfesten Rührbehältern oder in einer Rührbehälterkaskade durch. In einer besonders bevorzugten Ausführungsform werden Rohrreaktoren eingesetzt, in denen das Produktgemisch aus der Iminierung in Sumpf- oder Rieselfahrweise über ein fest angeordnetes Katalysatorbett geleitet wird.
   Der Reaktoraustrag enthält noch nicht vollständig umgesetzte Komponenten, wie z. B. das Aminonitril, welches von IPDA destillativ kaum abtrennbar ist.
c) Der aus b) erhaltene Reaktionsaustrag wird in eine dritte Stufe in Gegenwart von Wasserstoff und Ammoniak bei 110 bis 160°C und 50 bis 300 bar, bevorzugt 100 bis 250 bar, umgesetzt. Zweckmäßigerweise wählt man Ammoniak und Wasserstoffangebot, wie es sich im Reaktoraustritt der Stufe b) einstellt.
   Der Reaktor der Stufe c) kann deutlich kleiner als der Reaktor aus Stufe b) sein.
   Nach der Hydrierung wird überschüssiges Ammoniak gegebenenfalls unter Druck abgetrennt. Das so erhaltene 3-Aminomethyl-3,5,5-trimethyl-cyclohexylamin läßt sich durch fraktionierende Destillation isolieren.
   Bei der Hydrierung können prinzipiell alle gängigen Hydrierkatalysatoren eingesetzt werden, die Nickel, Cobalt, Eisen, Kupfer, Ruthenium oder andere Edelmetalle der VIII. Nebengruppe des Periodensystems enthalten. Bevorzugt verwendet man Ruthenium-, Cobalt- oder Nickel-Katalysatoren. Besonders bevorzugt sind Ruthenium- und Cobalt-Katalysatoren. Die katalytisch aktiven Metalle können als Vollkontakte oder auf Trägern eingesetzt werden. Als solche Träger kommen z.B. Aluminiumoxid, Titandioxid, Zirkondioxid, Zinkoxid oder Magnesiumoxid/Aluminiumoxide in Frage, bevorzugt werden Träger mit basischen Komponenten, wie Oxide und Hydroxide von Alkali- und Erdalkalimetallen. Besonders bevorzugt sind Vollkontakte, wie sie z.B. aus DE-A-43 25 847 bekannt sind, die basische Komponenten wie Oxide oder Hydroxide von Alkali und Erdalkalimetallen enthalten. Die basische Komponente kann gegebenenfalls auch während des Hydrierprozesses zugeführt werden, z.B. als Lösung von Alkali- oder Erdalkalihydroxiden in Wasser.

### Beispiele

### Beispiel 1

Die Apparatur bestand aus drei hintereinandergeschalteten Reaktoren. Der erste Reaktor war mit 240 l gamma-Aluminiumoxid-Strängen (4 mm-Stränge) gefüllt, der zweite Reaktor mit 600 l eines reduzierten Cobalt-Katalysators (90 % Kobalt mit 5 % Mangan und 1,9 % Natrium) in Form von 4 mm-Strängen und der dritte Reaktor mit 200 l eines reduzierten Cobalt-Katalysators (90 % Kobalt mit 5 % Mangan und 1,9 % Natrium) in Form von 4 mm-Strängen.

Bei einem Druck von 250 bar pumpte man in den ersten Reaktor stündlich 160 l IPN und 760 l Ammoniak, vor dem zweiten Reaktor führte man 250 l Ammoniak und 250 ml 2 %ige wäßrige Natronlauge sowie 220 Nm³ Wasserstoff hinzu. Nach dem zweiten Reaktor wurde ein Großteil des Wasserstoffs in einem Hochdruckabscheider abgetrennt und rückgeführt und die flüssige Phase nach Zugabe von 50 Nm³ Wasserstoff durch den dritten Reaktor gepumpt. Die Temperaturen betrugen im Iminierungsreaktor 90°C, im ersten Hydrierreaktor 75°C (Eintrittstemperatur) bis 100°C (Austrittstemperatur) und im zweiten Hydrierreaktor 140°C. Nach dem zweiten Hydrierreaktor enthielt das Reaktionsgemisch nach Abtrennen von Ammoniak laut gaschromatographischer Analyse (ohne Wasser) 96 % IPDA und 1,3 % Azabicyclooctan. Der Aminonitril-Anteil lag unter 200 ppm, der Anteil an cis-IPDA betrug 70 %. Nach dem ersten Reaktor waren neben 3,5 % Aminonitril noch weitere ungesättigte Komponenten mit einem Anteil von 3 bis 6 % enthalten.

### Vergleichsbeispiel A: (nach EP-A-449 089, analog Beispiel Nr.: 4)

Die Apparatur bestand aus zwei Reaktoren; der erste Reaktor war mit 170 l gamma-Aluminiumoxid-Strängen (4 mm-Stränge) gefüllt, der zweite Reaktor mit 330 l eines reduzierten Cobalt-Katalysators (90 % Kobalt mit 5 % Mangan und 1,9 % Natrium) in Form von 4 mm-Strängen.

Bei einem Druck von 250 bar wurden stündlich in die beiden hintereinandergeschalteten Reaktoren 72 l IPN und 630 l Ammoniak gepumpt. Zusätzlich in den Hydrierreaktor gab man 250 ml/h einer 2 %igen wäßrigen Natronlauge sowie 150 Nm³/h Wasserstoff. Die Temperatur im Iminierreaktor betrug 90°C, die Eintrittstemperatur im Hydrierreaktor 115°C, die Austrittstemperatur 140°C. Der Hydrieraustrag enthielt neben Ammoniak und Wasser laut gaschromatographischer Analyse 97 % IPDA, 1 % 1,3,3-Trimethyl-6-azabi-cyclo[3,2,1]octan sowie < 200 ppm Aminonitril. Der cis-Anteil im IPDA betrug 60 %.

Dosierte man nur 150 ml/h 2 %ige Natronlauge, sank der IPDA-Gehalt auf 92,4 %, der Anteil des 1,3,3-Trimethyl-6-azabicyclooctans stieg auf 4,5 %, der cis-Anteil im IPDA auf 68 %.

Reduzierte man die Hydriertemperatur, so stieg in beiden Fällen das Aminonitril auf Werte > 1000 pm.

## Patentansprüche

1. Verfahren zur Herstellung von 3-Aminomethyl-3,5,5-trimethylcyclohexylamin aus 3-Cyano-3,5,5-trimethyl-cyclohexanon, dadurch gekennzeichnet, daß man in drei räumlich voneinander getrennten Reaktionsräumen
a) das 3-Cyano-3,5,5-trimethyl-cyclohexanon in einem ersten Reaktionsraum mit überschüssigem Ammoniak an aciden Metalloxidkatalysatoren bei Temperaturen von 20 bis 150°C und Drücken von 50 bis 300 bar umsetzt,
b) die entstandenen Reaktionsprodukte in einem zweiten Reaktionsraum mit Wasserstoff in Gegenwart von überschüssigem Ammoniak an Hydrierkatalysatoren gegebenenfalls mit basischen Komponenten oder auf neutralen oder basischen Trägern bei Temperaturen von 50 bis 100°C und Drücken von 50 bis 300 bar hydriert und
c) die entstandenen Reaktionsprodukte in einem dritten Reaktionsraum in Gegenwart von Wasserstoff an Hydrierkatalysatoren gegebenenfalls mit basischen Komponenten oder auf neutralen oder basischen Trägern bei Temperaturen von 110 bis 160°C und Drücken von 150 bis 300 bar hydriert.

2. Verfahren zur Herstellung von 3-Aminomethyl-3,5,5-trimethylcyclohexylamin nach Anspruch 1, dadurch gekennzeichnet, daß man als Hydrierkatalysatoren cobalt-, nickel-, ruthenium- und/oder andere edelmetallhaltige Katalysatoren einsetzt.

3. Verfahren zur Herstellung von 3-Aminomethyl-3,5,5-trimethylcyclohexylamin nach Anspruch 1, dadurch gekennzeichnet, daß die Hydrierkatalysatoren als basische Komponenten Oxide oder Hydroxide von Alkali- oder Erdalkalimetallen enthalten.

## Claims

1. A process for the preparation of 3-aminomethyl-3,5,5-trimethylcyclohexylamine from 3-cyano-3,5,5-trimethylcyclohexanone in three spatially separated reaction spaces, which comprises
a) reacting the 3-cyano-3,5,5-trimethylcyclohexanone with excess ammonia on acidic metal-oxide catalysts in a first reaction space at from 20 to 150°C and at from 50 to 300 bar,
b) hydrogenating the resultant reaction products using hydrogen in a second reaction space in the presence of excess ammonia on hydrogenation catalysts, with or without basic components or on neutral or basic supports at from 50 to 100°C and at from 50 to 300 bar, and
c) hydrogenating the resultant reaction products in the presence of hydrogen in a third reaction space on hydrogenation catalysts, with or without basic components or on neutral or basic supports at from 110 to 160°C and at from 150 to 300 bar.

2. A process for the preparation of 3-aminomethyl-3,5,5-trimethylcyclohexylamine as claimed in claim 1, wherein the hydrogenation catalysts employed contain cobalt, nickel, ruthenium and/or other noble metals.

3. A process for the preparation of 3-aminomethyl-3,5,5-trimethylcyclohexylamine as claimed in claim 1, wherein the basic components in the hydrogenation catalysts are oxides or hydroxides of alkali metals or alkaline earth metals.

## Revendications

1. Procédé de préparation de 3-aminométhyl-3,5,5-triméthylcyclohexylamine à partir de 3-cyano-3,5,5-triméthylcyclonexanone, caractérisé en ce que l'on procède, dans trois zones réactionnelles séparées les unes des autres, à
a) la mise en réaction de la 3-cyano-3,5,5-triméthylcyclohexanone dans une première zone réactionnelle avec de l'ammoniac en excès, en présence de catalyseurs acides à base d'oxydes métalliques, à des températures de 20-150°C et sous des pressions de 50-300 bar,
b) l'hydrogénation du produit réactionnel résultant dans une deuxième zone réactionnelle avec de l'hydrogène en présence d'ammoniac en excès, sur des catalyseurs d'hydrogénation avec d'éventuels composants basiques ou sur des supports neutres ou basiques, à des températures de 50-100°C et sous des pressions de 50-300 bar et
c) l'hydrogénation du produit réactionnel résultant dans une troisième zone réactionnelle en présence d'hydrogène, sur des catalyseurs d'hydrogénation avec d'éventuels composants basiques ou sur des supports neutres ou basiques, à des températures de 110-160°C et sous des pressions de 150-300 bar.

2. Procédé de préparation de 3-aminométhyl-3,5,5-triméthylcyclohexylamine selon la revendication 1, caractérisé en ce que l'on utilise, en tant que catalyseurs d'hydrogénation, des catalyseurs contenant du cobalt, du nickel, du ruthénium et/ou d'autres métaux précieux.

3. Procédé de préparation de 3-aminométhyl-3,5,5-triméthylcyclohexylamine selon la revendication 1, caractérisé en ce que les catalyseurs d'hydrogénation contiennent, en tant que composants basiques, des oxydes ou des hydroxydes de métaux alcalins ou alcalino-terreux.
